# FASCICULE DE BREVET EUROPEEN

(11) **EP 1 833 269 B1**
(45) Date de publication et mention de la délivrance du brevet: **12.05.2010**
(21) Numéro de dépôt: 06300204.2
(22) Date de dépôt: 06.03.2006
(51) Int. Cl.: H04W 4/12

(54) **Contrôle de condition pour la transmission des messages**
Bedingungssteuerung zum Übertragen von Nachrichten
Condition control for transmitting messages

(43) Date de publication de la demande: 12.09.2007
(73) Titulaire: Alcatel Lucent, 75008 Paris (FR)
(72) Inventeur: Robinson, Julien, 75014 Paris (FR); Boussard, Mathieu, 77930 Chailly-en-Bière (FR); Leclerc, Denis, 91430 Igny (FR); Bataille, Fabien, 92160 Antony (FR); Hebbar, Abdelkrim, 91400 Orsay (FR); Mongazon-Cazavet, Bruno, 91240 St Michel-sur-Orge (FR)
(74) Mandataire: Korakis-Ménager, Sophie

(56) Documents cités:
- US-A1- 2002 173 304
- US-A1- 2005 089 019

## Description

L'invention concerne le domaine des systèmes, des dispositifs et des procédés de contrôle de condition pour la transmission des messages à leurs destinataires. La condition ou au moins l'une des conditions est de préférence liée au contexte de l'utilisateur récepteur du message.

Selon un premier art antérieur, par exemple décrit dans la demande de brevet américain US 2005/0186978, il est connu un dispositif de contrôle de condition, mais ce contrôle est purement interne ; en effet, il ne fait pas appel à un quelconque serveur de données externe au dispositif de contrôle.

Selon un deuxième art antérieur, par exemple décrit dans la demande US 2002/0173304, il est connu un dispositif de transmission de message d'alerte de serveurs de données externes au dispositif de transmission vers des terminaux ; mais le contrôle de condition pour la transmission des messages d'alerte ne fait pas appel à une information obtenue de la part d'un serveur de données externe qui serait quant à elle distincte du message d'alerte lui-même.

Un autre exemple d'art antérieur se trouve dans US 2005/0089019.

L'invention propose de pouvoir réaliser un contrôle de condition avant transmission d'un message à un destinataire, à l'aide d'au moins une information obtenue d'un serveur de données externe au dispositif de contrôle et au terminal émetteur, l'information externe utilisée pour le contrôle ne provenant ainsi ni du message devant être contrôlé ni du dispositif de contrôle lui-même. Il s'ensuit une plus grande flexibilité et une plus grande richesse dans les types de condition qui peuvent être attachés à un message pour permettre sa transmission.

L'invention concerne à la fois le système de contrôle, le dispositif de contrôle et le procédé de contrôle. Le système de contrôle et le dispositif de contrôle peuvent être implémentés de deux manières distinctes, à l'aide d'un dispositif de contrôle distinct du ou des systèmes de messagerie ou bien à l'aide d'au moins un dispositif de contrôle intégré à un système de messagerie sous forme d'une couche applicative dudit système de messagerie. Chaque système de messagerie, s'il y en a plusieurs, pourrait alors avoir sa propre couche applicative de contrôle.

Selon l'invention, il est prévu un système comprenant : un premier terminal ; un deuxième terminal ; au moins un premier système de messagerie capable de transmettre, vers le deuxième terminal, au moins le corps de message d'un message issu du premier terminal ; au moins un dispositif de contrôle d'au moins une condition, au moins une fois, ledit dispositif de contrôle permettant au premier système de messagerie de transmettre au deuxième terminal au moins le corps de message dudit message si le résultat de contrôle de condition est positif, ledit dispositif de contrôle empêchant le premier système de messagerie de transmettre au deuxième terminal au moins le corps de message dudit message si le résultat de contrôle de condition est négatif au moins pendant le temps où le résultat de contrôle de condition reste négatif ; caractérisé en ce que le système comprend aussi : au moins un serveur de données externe au dispositif de contrôle ; au moins une liaison de communication au travers du réseau Internet entre d'une part le serveur externe ou au moins l'un des serveurs externes et d'autre part le dispositif de contrôle ; et en ce que le dispositif de contrôle de condition est adapté pour : stocker un message envoyé par le premier terminal ; recevoir au moins une information de la part d'au moins un serveur externe ; effectuer au moins en partie le contrôle de condition relatif audit message stocké à l'aide de l'information reçue du serveur externe.

Selon l'invention, il est aussi prévu un système comprenant : un premier terminal ; un deuxième terminal ; au moins un premier système de messagerie capable de transmettre, du premier terminal vers le deuxième terminal, au moins le corps de message d'un message, et présentant une couche applicative de contrôle d'au moins une condition au moins une fois ; ladite couche applicative de contrôle permettant au premier système de messagerie de transmettre au deuxième terminal au moins le corps de message dudit message si le résultat de contrôle de condition est positif, ladite couche applicative de contrôle empêchant le premier système de messagerie de transmettre au deuxième terminal au moins le corps de message dudit message si le résultat de contrôle de condition est négatif au moins pendant le temps où le résultat de contrôle de condition reste négatif ; caractérisé en ce que le système comprend aussi : au moins un serveur de données externe au premier système de messagerie ; au moins une liaison de communication au travers du réseau Internet entre d'une part le serveur externe ou au moins l'un des serveurs externes et d'autre part le premier système de messagerie ; et en ce que la couche applicative de contrôle de condition est adaptée pour : stocker, ou faire stocker par le premier système de messagerie, un message envoyé par le premier terminal ; recevoir au moins une information de la part d'au moins un serveur externe ; effectuer au moins en partie le contrôle de condition relatif audit message stocké à l'aide de l'information reçue du serveur externe.

Selon l'invention, il est également prévu un dispositif de contrôle d'au moins une condition, au moins une fois, adapté pour : permettre à un système de messagerie de transmettre à un terminal au moins le corps de message d'un message envoyé par un autre terminal si le résultat de contrôle de condition est positif ; empêcher un système de messagerie de transmettre à un terminal au moins le corps de message d'un message envoyé par un autre terminal si le résultat de contrôle de condition est négatif au moins pendant le temps où le résultat de contrôle de condition reste négatif ; caractérisé en ce que le dispositif de contrôle est aussi adapté pour : être relié au travers du réseau Internet à au moins un serveur de données externe au dispositif de contrôle ; et en ce que le dispositif de contrôle de condition est également adapté pour : stocker un message envoyé par un terminal ; recevoir au moins une information de la part d'au moins un serveur externe ; effectuer au moins en partie le contrôle de condition relatif audit message stocké à l'aide de l'information reçue du serveur externe.

Selon l'invention, il est encore prévu une couche applicative de contrôle d'au moins une condition, au moins une fois, destinée à être intégrée dans un système de messagerie, adaptée pour : permettre à un système de messagerie de transmettre à un terminal au moins le corps de message d'un message envoyé par un autre terminal si le résultat de contrôle de condition est positif ; empêcher un système de messagerie de transmettre à un terminal au moins le corps de message d'un message envoyé par un autre terminal si le résultat de contrôle de condition est négatif au moins pendant le temps où le résultat de contrôle de condition reste négatif ; caractérisé en ce que la couche applicative de contrôle est aussi adaptée pour : être reliée au travers du réseau Internet à au moins un serveur de données externe à la couche applicative de contrôle ; et en ce que la couche applicative de contrôle de condition est également adaptée pour : stocker, ou faire stocker par un système de messagerie, un message envoyé par un terminal ; recevoir au moins une information de la part d'au moins un serveur externe ; effectuer au moins en partie le contrôle de condition relatif audit message stocké à l'aide de l'information reçue du serveur externe.

Selon l'invention, il est enfin prévu un procédé de contrôle d'au moins une condition, au moins une fois, comprenant : une étape de contrôle de condition qui, en cas de résultat de contrôle de condition positif, permet à un système de messagerie de transmettre à un terminal au moins le corps de message d'un message envoyé par un autre terminal, et qui, en cas de résultat de contrôle de condition négatif, empêche un système de messagerie de transmettre à un terminal au moins le corps de message d'un message envoyé par un autre terminal au moins pendant le temps où le résultat de contrôle de condition reste négatif ; caractérisé en ce que le procédé de contrôle comprend aussi : une étape de stockage d'un message envoyé par un terminal ; une étape de réception d'au moins une information de la part d'au moins un serveur de données externe par l'intermédiaire d'une communication au travers du réseau Internet ; et en ce que : l'étape de contrôle de condition relatif audit message stocké est réalisée au moins en partie à l'aide de l'information reçue du serveur externe.

L'invention sera mieux comprise et d'autres particularités et avantages apparaîtront à l'aide de la description ci-après et des dessins joints, donnés à titre d'exemples, où :
- la figure 1 représente schématiquement un exemple de réalisation préférentielle d'un système intégrant un dispositif de contrôle de condition, des terminaux, des systèmes de messagerie, des serveurs de données ;
- la figure 2 représente schématiquement un exemple de réalisation préférentielle d'un message dans lequel le champ condition est intégré au travers d'une extension du format du message.

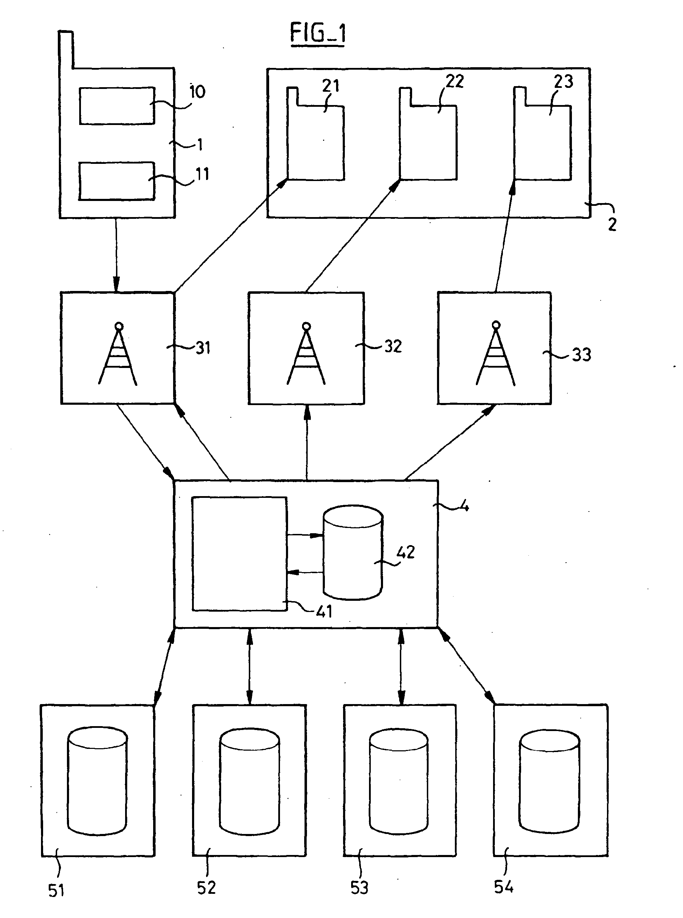

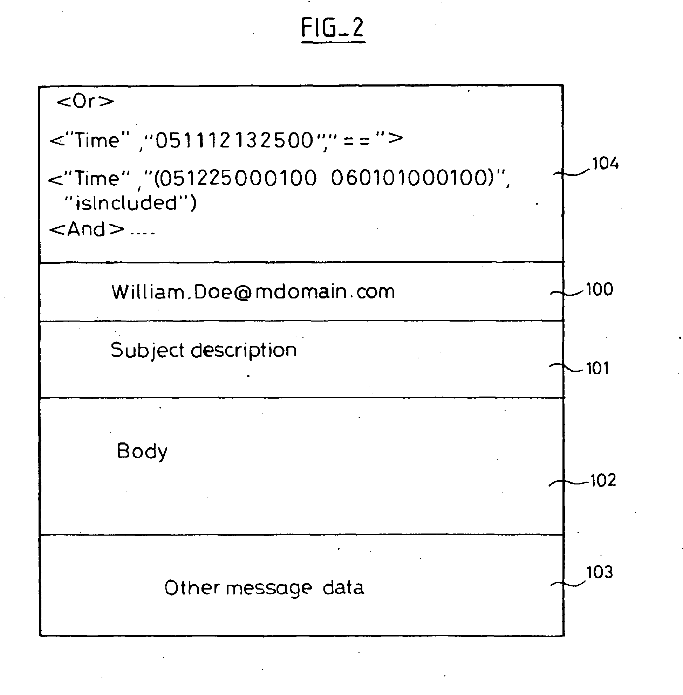

Sur les figures 1 et 2, les flèches symbolisent des échanges d'informations. La figure 2 ne représente qu'un exemple de message à condition présentant un format ayant été étendu par addition d'un champ condition. Le système de contrôle de condition faisant appel à au moins un serveur de données externe, ainsi que le dispositif de contrôle et le procédé de contrôle associés, peuvent aussi être utilisés pour contrôler un message encapsulé dans un élément d'encapsulation contenant le champ condition devant être contrôlé. Ils peuvent même encore être utilisés pour contrôler un message classique à l'aide de conditions implémentées dans le réseau de communication au travers de choix utilisateur uniformes pour tous les messages envoyés par lesdits utilisateurs, mais cette implémentation est nettement moins intéressante, car elle manque de souplesse et de simplicité pour la fixation des conditions par l'utilisateur émetteur du message.

La figure 1 représente schématiquement un exemple de réalisation préférentielle d'un système intégrant un dispositif de contrôle de condition, des terminaux, des systèmes de messagerie, des serveurs de données. Un premier terminal 1, appelé dans la suite terminal émetteur, est susceptible d'envoyer un message 10 en lui attachant au moins une condition 11. Le terminal 1 est de préférence un terminal utilisateur, c'est-à-dire susceptible d'envoyer un message ayant n'importe quel contenu à n'importe quel destinataire de son choix. Le message 10 est envoyé à destination d'un utilisateur récepteur 2. Cet utilisateur récepteur 2 possède au moins un terminal récepteur 21, il peut en posséder plusieurs, par exemple également des terminaux récepteurs 22 et 23. Le message 10 est envoyé généralement à destination d'un terminal récepteur, par exemple le terminal récepteur 21 ; toutefois, ce message 10 pourra éventuellement dans certaines conditions être redirigé vers un autre des terminaux récepteurs de l'utilisateur récepteur 2, soit vers le terminal récepteur 22 soit vers le terminal récepteur 23. Un ou plusieurs systèmes de messagerie, ici les systèmes de messagerie 31, 32 et 33, peuvent transmettre des messages entre certains terminaux. Le message 10 est envoyé par le terminal émetteur 1 au travers d'au moins un premier système de messagerie, par exemple le système de messagerie 31, capable de transmettre, vers le deuxième terminal récepteur, par exemple le terminal récepteur 21, au moins le corps de message d'un message 10 issu du premier terminal émetteur 1. Dans le cas le plus simple, le système de messagerie 31 achemine le message 10 du terminal émetteur 1 jusqu'au terminal récepteur 21. Il est également possible que le message 10 soit envoyé au travers du système de messagerie 31 à destination de l'utilisateur récepteur 2, mais qu'avant d'arriver au récepteur 21, il soit redirigé au travers d'un autre système de messagerie, par exemple le système de messagerie 32, vers un autre terminal récepteur, par exemple le terminal récepteur 22. Dans ce cas, le message 10 transmis par le système de messagerie par exemple 32 vers le terminal récepteur 22 est issu du terminal émetteur 1, mais il n'a pas été émis à l'origine au travers du système de messagerie 32, mais au travers d'un autre système de messagerie, le système de messagerie 31. Dans un cas simple, le message 10 peut être transmis au terminal récepteur en entier. Au moins le corps de message du message 10 devant être transmis, car c'est la partie que l'utilisateur émetteur destinait spécifiquement à la lecture de l'utilisateur récepteur, il peut arriver que seule une partie du message 10 soit transmise, par exemple sans la condition 11 de manière à ce que l'utilisateur récepteur 2 ne puisse pas en prendre connaissance. Ici, chaque terminal récepteur a été représenté comme couplé à un système de messagerie différent, mais plusieurs terminaux récepteurs peuvent aussi être couplés au même système de messagerie. Le terminal émetteur 1 pour un message 10 peut éventuellement devenir terminal récepteur pour un autre message à condition, mais pas nécessairement. Le terminal récepteur du message 10 peut éventuellement aussi devenir terminal émetteur pour un autre message à condition, mais pas nécessairement. Les terminaux, émetteur comme récepteur, peuvent être par exemple un téléphone fixe ou mobile, un ordinateur portable, un PDA (« Personal Digital Assistant » en langue anglaise).

Au moins un dispositif de contrôle 4 de condition va effectuer un contrôle de condition qui présentera un résultat positif ou négatif. S'il n'y a qu'une condition à vérifier, si cette condition est vérifiée, le résultat de contrôle de condition est positif, tandis que si cette condition n'est pas vérifiée, le résultat de contrôle est négatif. S'il y a une combinaison logique de plusieurs conditions élémentaires à vérifier, si cette combinaison logique de plusieurs conditions élémentaires est vérifiée dans son ensemble, le résultat de contrôle de condition est positif, tandis que si cette combinaison logique de plusieurs conditions élémentaires n'est pas vérifiée dans son ensemble, le résultat de contrôle est négatif. Une combinaison logique de plusieurs conditions élémentaires peut être vraie dans son ensemble même si toutes les conditions élémentaires ne sont pas vraies ; cela dépend des opérateurs logiques situés entre les conditions élémentaires. Dans le cas où le résultat de contrôle est positif, le dispositif de contrôle permet au système de messagerie chargé de transmettre le message 10, ou au moins le corps de message du message 10, vers le terminal récepteur, de le transmettre effectivement. Dans le cas où le résultat de contrôle est négatif, le dispositif de contrôle empêche le système de messagerie chargé de transmettre le message 10, ou au moins le corps de message du message 10, vers le terminal récepteur, de le transmettre effectivement, au moins pendant le temps que le résultat de condition reste négatif. Plus simplement, le dispositif de contrôle 4 permet au message 10 ou l'empêche de continuer à être acheminé vers le terminal récepteur auquel il était destiné ou vers le terminal récepteur vers lequel il devait être redirigé, selon le résultat du contrôle de condition. En cas de résultat positif, le message 10 est transmis. En cas de résultat négatif, le dispositif de contrôle peut soit supprimer le message 10, soit réeffectuer plus tard le contrôle de condition une ou plusieurs fois, par exemple périodiquement, jusqu'à ce que le contrôle de condition devienne positif, ou encore effectuer une ou plusieurs fois le contrôle de condition, et supprimer le message 10 à partir d'un ou de plusieurs résultats négatifs, ou encore à l'issue d'une temporisation avantageusement choisie par l'utilisateur émetteur du terminal émetteur 1. Le dispositif de contrôle présente préférentiellement deux parties, une partie 41 qui effectue le contrôle de condition et une partie 42 qui stocke les messages présentant au moins une condition devant être vérifiée.

Le contrôle de condition est effectué par le dispositif de contrôle 4 à l'aide d'au moins un serveur de données externe. Ici, le dispositif de contrôle 4 peut avoir accès à plusieurs serveurs de données externes pour l'aider à effectuer le contrôle, qui sont les serveurs de données externes 51 à 54. Un serveur de données externe est un serveur qui est externe au dispositif de contrôle 4, c'est-à-dire qu'il existe au moins une liaison de communication au travers du réseau Internet entre d'une part le serveur externe et d'autre part le dispositif de contrôle 4. Le dispositif de contrôle 4 stocke le message 10 envoyé par le terminal émetteur 1, au moins le temps de pouvoir effectuer le contrôle de condition. Le dispositif de contrôle 4 reçoit au moins une information de la part d'au moins un serveur externe. La réception d'information peut arriver après le stockage du message si le dispositif de contrôle 4 le demande après réception du message. Dans le cas où le serveur externe envoie régulièrement des informations mises à jour au dispositif de contrôle 4, l'information nécessaire peut déjà être disponible pour le dispositif de contrôle 4 lorsqu'il reçoit le message 10. Dans le cas le plus simple, il reçoit une information de la part d'un serveur externe. Dans des cas plus complexes, il peut recevoir plusieurs informations de la part d'un serveur, ou une ou plusieurs informations de la part de plusieurs serveurs. Le dispositif de contrôle 4 effectue au moins en partie le contrôle de condition relatif audit message 10 stocké à l'aide de l'information ou des informations reçue(s) du serveur externe ou des serveurs externes. Le contrôle s'effectue au moins en partie à l'aide de cette ou de ces informations reçue(s) de la part du serveur externe ou des serveurs externes, car le contrôle de condition peut aussi faire intervenir une ou plusieurs autres informations, comme par exemple une information interne au dispositif de contrôle 4, ou comme une information contenue dans le message 10. La ou au moins l'une des conditions est de préférence obligatoire au moins pendant un certain temps ; mais il est possible qu'une ou certaines conditions, ou même toutes, soient facultatives dans le sens où leur vérification n'est pas nécessaire pour la transmission du message si le résultat de contrôle ne peut être effectué, faute d'information.

Dans une autre réalisation non représentée sur la figure 1, le dispositif de contrôle est en fait intégré à un système de messagerie et se présente sous la forme d'une couche applicative de contrôle de condition, auquel cas le changement de système de messagerie en cours d'acheminement du terminal émetteur vers le terminal récepteur n'est pas possible. Le serveur de données est alors externe au système de messagerie, c'est-à-dire qu'il existe au moins une liaison de communication au travers du réseau Internet entre d'une part le serveur externe et d'autre part le système de messagerie. La couche applicative de contrôle de condition, soit stocke elle-même soit fait stocker par le système de messagerie, le message 10 envoyé par le terminal émetteur 1, jusqu'à ce que le contrôle de condition soit effectué.

De préférence, la condition ou au moins l'une des conditions à contrôler est relative au terminal récepteur. Ce peut être aussi une condition relative plus généralement à l'utilisateur récepteur lui-même.

De préférence, le système comprend au moins un autre système de messagerie capable de transmettre un message d'un type différent de celui du premier système de messagerie. Par exemple en effet, alors que le message 10 est à destination de l'utilisateur récepteur 2, et plus précisément du terminal récepteur 21 couplé au même système de messagerie 31 que le terminal émetteur 1, ce message 10, ou au moins le corps de message de ce message 10, peut être redirigé au travers d'un autre système de messagerie 33, à destination d'un terminal récepteur 23 qui lui est couplé. Différents exemples de type de message peuvent être des SMS (« Short Message Service » en langue anglaise), des MMS (« Multimedia Message Service» en langue anglaise), des mails, des messages vocaux avec un champ condition qui serait vocal aussi, etc ...

Dans le cas de la transmission entre un terminal émetteur utilisant un système de messagerie (par exemple le mail) et un terminal récepteur utilisant un autre système de messagerie (par exemple le SMS), il peut y avoir une transcription d'un format à l'autre pour permettre la transmission du message.

De préférence, soit à la demande du terminal émetteur 1, soit à l'expiration d'une période prédéterminée, en cas de résultat de contrôle négatif, un message d'échec est renvoyé vers le terminal émetteur 1. Ainsi, dans le premier cas, l'utilisateur émetteur sait à quoi s'en tenir sur le sort de son message 10, et dans le deuxième cas, en cas de conditions trop difficiles à réaliser, on évite que le dispositif de contrôle 4, et plus particulièrement la partie 42 stockant les messages à condition, ne devienne encombré par de trop nombreux messages présentant des conditions jamais réalisées, car une fois le message d'échec envoyé et l'utilisateur émetteur averti, le dispositif de contrôle peut alors supprimer le message 10.

De préférence, le système comprend au moins un autre serveur de données externe pouvant être consulté lors du contrôle de condition, pour le contrôle d'une même condition. Ceci améliore la fiabilité du système, car si un serveur externe est déficient, le contrôle de condition peut tout de même être effectué grâce à ce serveur de rechange qui ne coûte rien au système car il existe déjà dans le réseau Internet.

De préférence, la liaison vers le serveur externe est assurée par l'intermédiaire d'un abonnement auprès du serveur externe, ce qui permet au dispositif de contrôle d'être averti dès qu'une condition non vérifiée devient vérifiée. Sinon, il lui est nécessaire de réaliser soit de nombreuses consultations rapprochées, soit plusieurs consultations espacées avec le risque ou bien de ne transmettre le message que longtemps après la réalisation des conditions ou bien, s'il y a plusieurs conditions à satisfaire, que celles-ci ne soient jamais satisfaites simultanément pendant suffisamment longtemps pour aboutir à un résultat de contrôle de condition qui puisse être positif.

Le serveur externe est un serveur non contrôlé par le dispositif de contrôle 4. C'est un serveur classique qui appartient au réseau Internet et qui peut, au moins techniquement même si une autorisation peut parfois être nécessaire, être accédé par d'autres utilisateurs du réseau Internet. Plusieurs exemples de serveurs de données externes vont maintenant être cités. Le serveur externe ou au moins l'un des serveurs externes est par exemple une horloge. Un exemple de condition est de ne transmettre le message 10 que le lendemain de son envoi. Le serveur externe ou au moins l'un des serveurs externes est par exemple un serveur de localisation du terminal récepteur. Un exemple de condition est de ne transmettre le message 10 que lorsque le terminal récepteur est dans la ville de New York. Les horloges et les serveurs de localisation sont les types de serveur les plus intéressants en pratique. D'autres serveurs sont toutefois possibles. Le serveur externe ou au moins l'un des serveurs externes est par exemple un capteur biométrique associé au terminal récepteur. Une information de niveau de stress de l'utilisateur récepteur permet par exemple de ne lui transmettre un message que lorsqu'il est en état psychologique de le recevoir. Le serveur externe ou au moins l'un des serveurs externes est par exemple un serveur météo. Un exemple de condition est de ne transmettre le message que lorsqu'il y aura de la neige sur Paris. Le serveur externe ou au moins l'un des serveurs externes est une base de données psychologiques renseignée par l'utilisateur du terminal récepteur. Un exemple de condition est de ne transmettre le message 10 que lorsque l'utilisateur récepteur aura indiqué qu'il est à nouveau de bonne humeur. Une ou plusieurs conditions à contrôler peuvent également l'être de manière interne au dispositif de contrôle, à l'aide d'information(s) disponible(s) dans le dispositif de contrôle lui-même, mais au moins une condition à contrôler l'est à l'aide d'un serveur externe.

La figure 2 représente schématiquement un exemple de réalisation préférentielle d'un message dans lequel le champ condition est intégré au travers d'une extension du format du message. Le message comprend un corps de message 102, un champ émetteur non représenté ici car implicite et un champ récepteur 100. Ici, lors de la composition du message, ne sont représentés que les champs que l'utilisateur émetteur est susceptible de remplir, à savoir au moins le champ récepteur 100 et le corps de message 102. Le message peut aussi contenir d'autres champs facultatifs à remplir par l'émetteur s'il le désire, comme par exemple un champs sujet de message 101 ou un champs données complémentaires de message 103. Le message est destiné à être envoyé par un émetteur, utilisateur émetteur ou terminal émetteur, associé au champ émetteur vers un récepteur, utilisateur récepteur ou terminal récepteur, associé au champ récepteur.

Le message comprend aussi un champ condition 104 qui est destiné à être rempli par l'émetteur associé et qui peut contenir le ou les paramètres représentatifs d'au moins une condition élémentaire, notamment la condition 11 de la figure 1, qui est destinée à être vérifiée par un serveur associé au champ condition 104, notamment le dispositif de contrôle de la figure 1. Le serveur associé est distinct du récepteur associé ; en effet, le serveur associé n'est pas une partie du récepteur associé car le récepteur associé ne doit pas être susceptible de contrôler ce serveur associé ou de modifier des messages qu'il n'aurait pas émis. En effet, le récepteur associé ne doit pas pouvoir influer sur la ou les conditions posées par l'émetteur associé pour la transmission du message, ou en tous cas par contre la volonté de l'utilisateur émetteur. Le serveur associé est destiné à transmettre au moins le corps de message vers le récepteur associé si la condition élémentaire est vérifiée, ledit serveur associé étant destiné à ne pas transmettre le corps de message vers le récepteur associé si la condition élémentaire n'est pas vérifiée, au moins aussi longtemps que la condition élémentaire n'est pas vérifiée. En effet, lorsque la condition élémentaire n'est pas vérifiée, le contrôle de condition peut être préférentiellement effectué plusieurs fois, éventuellement périodiquement, en cas d'échec de la première fois. Ce réessai de contrôle de condition est effectué, soit jusqu'à ce que la condition élémentaire devienne vérifiée, soit avantageusement pendant un certain temps seulement après lequel le message peut être tout simplement supprimé par le serveur associé, un message d'échec issu du serveur associé signalant éventuellement à l'émetteur associé l'échec de la tentative de transmission du message.

Le champ condition 104 peut consister en le cas simple de ne nécessiter que la vérification d'une condition élémentaire. D'autres cas plus complexes sont bien sûr envisageables, comme le cas où le champ condition 104 peut contenir le ou les paramètres représentatifs d'au moins une combinaison logique de plusieurs conditions élémentaires qui est destinée à être vérifiée par un serveur associé au champ condition. Le serveur associé est alors destiné à transmettre au moins le corps de message vers le récepteur associé si la combinaison logique est vérifiée, ledit serveur associé étant destiné à ne pas transmettre le corps de message vers le récepteur associé si la combinaison logique n'est pas vérifiée. En cas de non-vérification de la combinaison logique, une ou plusieurs tentatives de réessai comme dans le cas de la condition élémentaire décrite ci-dessus restent possibles. Les opérateurs logiques entre conditions élémentaires peuvent de préférence être de plusieurs types différents, ils sont alors avantageusement explicités dans le champ condition 104, ils permettent ainsi plus de richesse et de flexibilité dans la constitution de la combinaison logique de conditions élémentaires. Le ou les opérateurs logiques pourraient aussi être implicites, soit s'il n'y en qu'un de possible, par exemple l'opérateur ET, soit si pour au moins une ou plusieurs conditions élémentaires, le type de chacune de ces conditions élémentaires commande un seul opérateur logique et pas un autre. D'autres exemples non limitatifs d'opérateurs logiques entre conditions élémentaires peuvent être, OU, OU exclusif, ET NE PAS, etc ... ou encore être plus évolués comme SAUF. Par exemple, transmettre le message après 14 heures ou bien avant si le récepteur associé est déjà à Paris. Par exemple transmettre au récepteur associé dès qu'il est à Paris sauf s'il neige ou sauf s'il est trop stressé.

L'exemple préférentiel de la figure 2 montre une implémentation dans laquelle le champ condition 104 est une extension du corps de message 102. En effet, le message présente un format dans lequel à la fois le corps de message 102 et le champ condition 104 sont intégrés. Cette implémentation, la plus simple à l'utilisation, nécessite tout de même un effort important à la mise en place, car il s'agit de définir un nouveau format qui rendra le message compréhensible et traitable par le serveur associé et qui devra permettre au système de messagerie de pouvoir continuer à transmettre le message. Dans le cas où le dispositif de contrôle de la figure 1 est intégré au système de messagerie sous la forme d'une couche applicative de ce système de messagerie, cette couche applicative pourra se charger de rendre le nouveau format compréhensible au système de messagerie sans devoir modifier celui-ci substantiellement. Une autre implémentation, non représentée à la figure 2, consiste à inclure le champ condition dans une encapsulation du corps de message. Cette implémentation permet une mise en place plus simple dans la mesure où on ne modifie pas le format d'origine du message, l'utilisation sera alors toutefois moins optimale.

De préférence, le champ condition 104 n'est jamais destiné à être transmis avec le corps de message 102 au récepteur associé. Ainsi la confidentialité de la volonté de l'utilisateur émetteur est conservée vis-à-vis de l'utilisateur récepteur. Il peut même être envisagé, selon le désir de l'utilisateur, que certaines conditions élémentaires soient masquées à l'utilisateur récepteur lors de la transmission du message tandis que d'autres conditions élémentaires restent visibles pour l'utilisateur récepteur. Dans ce cas aussi, seule une partie du message est réellement transmise.

De préférence, le champ condition comprend trois sous-champs pour au moins une, certaines ou toutes les conditions élémentaires, à savoir un type, une référence et un opérateur, et ladite condition élémentaire est vérifiée, lorsque la valeur du type vérifie la relation type opérateur référence, c'est-à-dire lorsque la comparaison entre le type et la référence par l'intermédiaire de l'opérateur donne un résultat positif. Ces sous-champs ne sont pas obligatoires, car comme vu plus haut, aussi bien notamment pour le type de condition que pour l'opérateur logique, ceux-ci peuvent être implicites et ne pas nécessiter d'apparaître dans le champ condition 104, notamment lors de la constitution du message par l'émetteur. Plus précisément, le champ condition 104 contient préférentiellement trois sous-champs, un premier sous-champ type condition, un deuxième sous-champ valeur condition, un troisième sous-champ opérateur. La vérification de condition élémentaire consiste à effectuer l'opération suivante : lors du contrôle, comparer la valeur effective au moment du contrôle ou à un instant proche, du type condition contenu dans le premier sous-champ d'une part et la valeur contenue dans le deuxième sous-champ d'autre part à l'aide de l'opérateur contenu dans le troisième sous-champ ; le résultat de cette comparaison entraînant la permission ou l'interdiction de transmission du message. Par exemple, soit le contenu des trois sous-champs suivants, « heure », « 14 », « supérieur à » ; la condition élémentaire correspondante sera vérifiée s'il est plus de 14 heures. Par exemple, soit le contenu des trois sous-champs suivants, « battements coeur », « 120 », « inférieur à » ; la condition élémentaire est vérifiée si le capteur biométrique associé au récepteur signale que le nombre des battements de coeur de l'utilisateur récepteur sont inférieurs à 120 battements par minute. Par exemple, soit le contenu des trois sous-champs suivants, « lieu », « Bavière », « inclusion» ; la condition élémentaire correspondante sera vérifiée si le récepteur se trouve à un endroit à l'intérieur de la région Bavière en Allemagne.

En résumé et de manière plus générale, le message comprenant un corps de message, un champ émetteur et un champ récepteur, comprend aussi un champ condition qui est rempli par l'émetteur et qui permet à un serveur distinct du récepteur, de transmettre ou de ne pas transmettre, au moins le corps de message vers le récepteur. C'est le résultat de l'évaluation du champ condition par ce serveur, qui peut être notamment le dispositif de contrôle de la figure 1 ou bien une couche applicative d'un système de messagerie, qui est à la base de la permission ou de l'interdiction de transmettre le message, ou plus précisément au moins une partie du message incluant le corps de message ou même à l'extrême réduite au corps de message.

Des exemples de type de condition vont maintenant être donnés. Par exemple, la condition élémentaire ou au moins l'une des conditions élémentaires est une condition de date. Un exemple de telle condition élémentaire est de transmettre le message dès le premier janvier 2006. Par exemple, la condition élémentaire ou au moins l'une des conditions élémentaires est une condition de localisation du récepteur associé. Un exemple de telle condition élémentaire est de transmettre le message dès que le récepteur sera dans le train à mi-chemin entre Paris et Vienne. Par exemple, la condition élémentaire ou au moins l'une des conditions élémentaires est une condition de biométrie issue d'un capteur biométrique associé au récepteur associé. Un exemple de telle condition élémentaire est transmettre tel message d'alerte pré-enregistré si le pouls de l'utilisateur émetteur n'est plus perceptible. Par exemple, la condition élémentaire ou au moins l'une des conditions élémentaires est une condition de météo. Un exemple de telle condition élémentaire est de transmettre le message dès la fin de la tempête sur tel village. Par exemple, la condition élémentaire ou au moins l'une des conditions élémentaires est une condition d'état psychologique issue d'une base de données renseignée par l'utilisateur du récepteur associé. Un exemple de condition élémentaire est de transmettre le message lorsque l'utilisateur récepteur indique sa volonté d'être à nouveau réceptif à des messages de nature humoristique.

Plusieurs langages peuvent être utilisés pour remplir le champ condition 104 du message. Par exemple le langage XML (« eXtensible Markup Language » en langue anglaise) permet de mieux structurer les données, ce qui est particulièrement utile lorsque le champ condition 104 comprend une combinaison de conditions élémentaires.

D'autres syntaxes sont possibles basées sur des règles logiques écrites dans des langages de plus haut niveau basé sur XML comme par exemple SWRL (« Semantic Web Rule Language » en langue anglaise).

Un exemple de rédaction du contenu d'un champ de condition à l'aide du langage XML est donné ci-après :

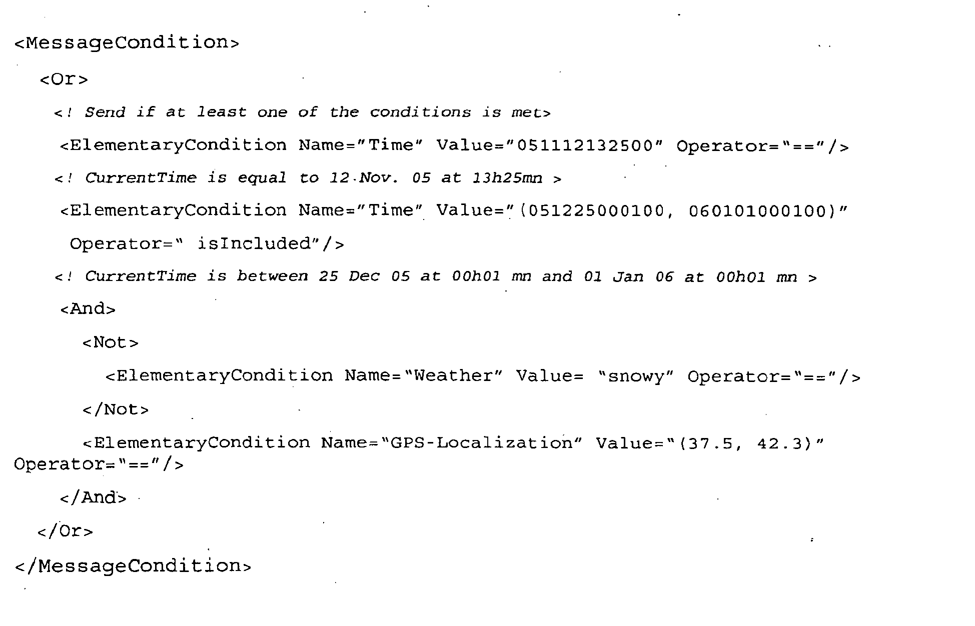

## Revendications

1. Système comprenant :
- un premier terminal (1) ;
- un deuxième terminal (21 ou 22 ou 23) ;
- au moins un premier système de messagerie (31 ou 32 ou 33) capable de transmettre, vers le deuxième terminal, au moins le corps de message d'un message (10) issu du premier terminal;
- au moins un dispositif de contrôle (4) d'au moins une condition (11), au moins une fois, ledit dispositif de contrôle permettant au premier système de messagerie de transmettre au deuxième terminal au moins le corps de message dudit message si le résultat de contrôle de condition est positif, ledit dispositif de contrôle empêchant le premier système de messagerie de transmettre au deuxième terminal au moins le corps de message dudit message si le résultat de contrôle de condition est négatif au moins pendant le temps où le résultat de contrôle de condition reste négatif ;
**caractérisé en ce que** le système comprend aussi :
- au moins un serveur (51 ou 52 ou 53 ou 54) de données externe au dispositif de contrôle ;
- au moins une liaison de communication au travers du réseau Internet entre d'une part le serveur externe ou au moins l'un des serveurs externes et d'autre part le dispositif de contrôle ;
et **en ce que** le dispositif de contrôle de condition est adapté pour :
- stocker un message qui a été envoyé par le premier terminal et auquel a été attachée au moins une condition ;
- recevoir au moins une information de la part d'au moins un serveur externe :
- effectuer au moins en partie le contrôle de la condition attachée audit message stocké à l'aide de l'information reçue du serveur externe.

2. Système selon la revendication 1, **caractérisé en ce que** le dispositif de contrôle (4) est intégré au premier système de messagerie.

3. Système selon l'une quelconque des revendications précédentes, **caractérisé en ce que** la condition ou au moins l'une des conditions à contrôler est relative au deuxième terminal ou à l'utilisateur du deuxième terminal,

4. Système selon l'une quelconque des revendications précédentes, **caractérisé en ce que** le système comprend au moins un autre serveur (51 ou 52 ou 53 ou 54) de données externe pouvant être consulté lors du contrôle de conditions, pour le contrôle d'une même condition.

5. Système selon l'une quelconque des revendications précédentes, **caractérisé en ce que** le système comprend au moins un autre système de messagerie (31 ou 32 ou 33) capable de transmettre un message d'un type différent de celui du premier système de messagerie.

6. Système selon l'une quelconque des revendications précédentes, **caractérisé en ce que** la liaison vers le serveur externe est assurée par l'intermédiaire d'un abonnement auprès du serveur externe.

7. Système selon l'une quelconque des revendications précédentes, **caractérisé en ce que**, soit à la demande du premier terminal soit à l'expiration d'une période prédéterminée, en cas de résultat de contrôle négatif, un message d'échec est renvoyé vers le premier terminal.

8. Système selon l'une quelconque des revendications précédentes, **caractérisé en ce que** le serveur externe ou au moins l'un des serveurs externes est une horloge.

9. Système selon l'une quelconque des revendications précédentes, **caractérisé en ce que** le serveur externe ou au moins l'un des serveurs externes est un serveur de localisation du deuxième terminal.

10. Système selon l'une quelconque des revendications précédentes, **caractérisé en ce que** le serveur externe ou au moins l'un des serveurs externes est un capteur biométrique associé au deuxième terminal.

11. système selon l'une quelconque des revendications précédentes, **caractérisé en ce que** le serveur externe ou au moins l'un des serveurs externes est un serveur météo.

12. Système selon l'une quelconque des revendications précédentes, **caractérisé en ce que** le serveur externe au au moins l'un des serveurs externes est une base de données psychologiques renseignée par l'utilisateur du deuxième terminal.

13. Dispositif de contrôle d'au moins une condition, au moins une fois, adapté pour :
- permettre à un système de messagerie de transmettre à une terminal au moins le corps de message d'un message envoyé par un autre terminal si le résultat de contrôle de condition est positif ;
- empêcher un système de messagerie de transmettre à un terminal au moins le corps de message d'un message envoyé par un autre terminal si le résultat de contrôle de condition est négatif au moins pendant le temps où le résultat de contrôle de condition reste négatif ;
**caractérisé en ce que** le dispositif de contrôle est aussi adapté pour :
- être relié au travers du réseau Internet à au moins un serveur de données externe au dispositif de contrôle ;
et **en ce que** le dispositif de contrôle de condition est également adapté pour :
- stocker un message qui a été envoyé par un terminal et auquel a été attachée au moins une condition ;
- recevoir au moins une information de la part d'au moins un serveur externe ;
- effectuer au moins en partie le contrôle de la condition attachée audit message stocké à l'aide de l'information reçue du serveur externe.

14. Procédé de contrôle d'au moins une conditions, au moins une fois, comprenant :
- une étape de contrôle de condition qui, en cas de résultat de contrôle de condition positif, permet à un système de messagerie de transmettre à un terminal au moins le corps de message d'un message envoyé par un autre terminal, et qui, en cas de résultat de contrôle de condition négatif, empêche un système de messagerie de transmettre à un terminal au moins le corps de message d'un message envoyé par un autre terminal au moins pendant le temps où le résultat de contrôle de condition reste négatif :
**caractérisé en ce que** le procédé de contrôle comprend aussi ;
- une étape de stockage d'un message qui a été envoyé par un terminal et auquel a été attachée au moins une condition ;
- une étape de réception d'au moins une information de la part d'au moins un serveur de données externe par l'intermédiaire d'une communication au travers du réseau Internet ;
et **en ce que** :
l'étape de contrôle de la condition attachée audit message stocké est réalisée au moins en partie à l'aide de l'information reçue du serveur externe.

## Claims

1. A system comprising:
- a first terminal (1);
- a second terminal (21 or 22 or 23);
- at least one first messaging system (31 or 32 or 33) able to send to the second terminal at least the message body for a message (10) from the first terminal;
- at least one device (4) for the control of at least one condition (11), at least once, said control device allowing the first messaging system to send the second terminal at least the message body of said message if the condition control result is positive, said control device preventing the first messaging system from sending the second terminal at least the message body of said message if the condition control result is negative, at least for the time for which the condition control result remains negative;
**characterized in that** the system also includes:
- at least one data server (51 or 52 or 53 or 54) external to the control device;
- at least one communication link through the Internet network between the external server or at least one of the external servers on the one hand, and the control device on the other hand;
and **in that** the condition control device is adapted to:
- store a message which has been sent by the first terminal and to which at least one condition has been attached;
- receive at least one item of information from at least one external server;
- carry out at least in part the control on the condition attached to said message stored using the information received from the external server.

2. System according to claim 1, **characterized in that** the control device (4) is integrated into the first messaging system.

3. System according to any of the previous claims, **characterized in that** the condition or at least one of the conditions to be controlled relates to the second terminal or to the user of the second terminal.

4. System according to any of the previous claims, **characterized in that** the system includes at least one other external data server (51 or 52 or 53 or 54) which can be consulted during the condition control, for the control of the same condition.

5. System according to any of the previous claims, **characterized in that** the system includes at least one other messaging system (31 or 32 or 33) able to send a message of a type other than that of the first messaging system.

6. System according to any of the previous claims, **characterized in that** the link to the external server is provided through a subscription to the external server.

7. System according to any of the previous claims, **characterized in that**, either on the request of the first terminal or on the expiry of a pre-determined period, in the event of a negative control result, a failure message is returned to the first terminal.

8. System according to any of the previous claims, **characterized in that** the external server or at least one of the external servers is a clock.

9. System according to any of the previous claims, **characterized in that** the external server or at least one of the external servers is a location server of the second terminal.

10. System according to any of the previous claims, **characterized in that** the external server or at least one of the external servers is a biometric sensor associated with the second terminal.

11. System according to any of the previous claims, **characterized in that** the external server or at least one of the external servers is a weather server.

12. System according to any of the previous claims, **characterized in that** the external server or at least one of the external servers is a database of psychological data completed by the user of the second terminal.

13. Device for the control of at least one condition, at least once, adapted to:
- allow a messaging system to send to a terminal at least the message body of a message sent by another terminal if the condition control result is positive;
- prevent a messaging system from sending to a terminal at least the message body of a message sent by another terminal if the condition control result is negative, at least for the time for which the condition control result remains negative;
**characterized in that** the control device is also adapted to:
- be connected through the Internet network to at least one data server external to the control device;
and **in that** the condition control device is also adapted to:
- store a message which has been sent by a terminal and to which at least one condition has been attached;
- receive at least one item of information from at least one external server;
- carry out at least in part the control on the condition attached to said message stored using the information received from the external server.

14. Method for the control of at least one condition, at least once, including:
- a condition control stage which, in the event of a positive condition control result, allows a messaging system to send to a terminal at least the message body of a message sent by another terminal and which, in the event of a negative condition control result, prevents a messaging system from sending to a terminal at least the message body of a message sent by another terminal at least for the time for which the condition control result remains negative;
**characterized in that** the control method also includes:
- a stage for storage of a message which has been sent by a terminal and to which at least one condition has been attached;
- a stage for reception of at least one item of information from at least one external data server through communication through the Internet network;
and **in that**:
the control stage for the condition attached to said stored message is carried out at least in part using the information received from the external server.

## Patentansprüche

1. System, umfassend:
- Ein erstes Endgerät (1)
- ein zweites Endgerät (21 oder 22 oder 23);
- mindestens ein erstes Nachrichtensystem (31 oder 32 oder 33), welches für die Übertragung zumindest des Körpers der Nachricht einer von dem ersten Endgerät gesendeten Nachricht (10) an das zweite Endgerät ausgelegt ist,
- mindestens eine Prüfvorrichtung (4) zur mindestens einmaligen Prüfung einer Bedingung (11), wobei die besagte Prüfvorrichtung das erste Nachrichtensystem befähigt, zumindest den Körper der besagten Nachricht an das zweite Endgerät zu übertragen, wenn das Ergebnis der Prüfung der Bedingung positiv ist, wobei die besagte Prüfvorrichtung das erste Nachrichtensystem daran hindert, zumindest den Körper der besagten Nachricht an das zweite Endgerät zu senden, wenn das Ergebnis der Prüfung der Bedingung negativ ist, zumindest während der Zeit, während welcher das Ergebnis der Prüfung der Bedingung negativ bleibt;
**dadurch gekennzeichnet, dass** das System weiterhin umfasst:
- mindestens einen prüfvorrichtungsexternen Datenserver (51 oder 52 oder 53 oder 54);
- mindestens eine Kommunikationsverbindung über das Internet zwischen dem externen Server oder zumindest einem der externen Server, einerseits, und der Prüfvorrichtung, andererseits;
und dass die Prüfvorrichtung dazu ausgelegt ist, die folgenden Schritte durchzuführen:
- Speichern der Nachricht, die von dem ersten Endgerät gesendet wurde und welcher mindestens eine Bedingung angehängt wurde;
- Empfangen mindestens einer Information von mindestens einem externen Server;
- Ausführen, mindestens teilweise, der Prüfung der der besagten gespeicherten Nachricht angehängten Bedingung anhand der von dem externen Server empfangenen Information.

2. System nach Anspruch 1, **dadurch gekennzeichnet, dass** die Prüfvorrichtung (4) im ersten Nachrichtensystem integriert ist.

3. System nach einem beliebigen der vorstehenden Ansprüche, **dadurch gekennzeichnet, dass** sich die zu prüfende Bedingung oder zumindest eine der zu prüfenden Bedingungen auf das zweite Endgerät oder auf den Benutzer des zweiten Endgeräts bezieht.

4. System nach einem beliebigen der vorstehenden Ansprüche, **dadurch gekennzeichnet, dass** das System mindestens einen weiteren externen Datenserver (51 oder 52 oder 53 oder 54), welcher anlässlich der Prüfung einer Bedingung befragt werden kann, für die Prüfung einer selben Bedingung umfasst.

5. System nach einem beliebigen der vorstehenden Ansprüche, **dadurch gekennzeichnet, dass** das System mindestens ein weiteres Nachrichtensystem (31 oder 32 oder 33) umfasst, welches dazu ausgelegt ist, eine Nachricht eines anderen Typs als den der Nachricht des ersten Nachrichtensystems zu übertragen.

6. System nach einem beliebigen der vorstehenden Ansprüche, **dadurch gekennzeichnet, dass** die Verbindung zum externen Server auf der Basis eines Abonnements bei dem externen Server gewährleistet wird.

7. System nach einem beliebigen der vorstehenden Ansprüche, **dadurch gekennzeichnet, dass**, wenn das Ergebnis der Prüfung negativ ist, entweder auf Anfrage des ersten Endgeräts oder nach Ablauf einer festgelegten Zeitspanne, eine Fehlermeldung an das erste Endgerät zurückgesendet wird.

8. System nach einem beliebigen der vorstehenden Ansprüche, **dadurch gekennzeichnet, dass** der externe Server oder zumindest einer der externen Server ein Taktgeber ist.

9. System nach einem beliebigen der vorstehenden Ansprüche, **dadurch gekennzeichnet, dass** der externe Server oder zumindest einer der externen Server ein Ortungsserver des zweiten Endgerätes ist.

10. System nach einem beliebigen der vorstehenden Ansprüche, **dadurch gekennzeichnet, dass** der externe Server oder zumindest einer der externen Server ein dem zweiten Endgerät zugeordneter biometrischer Sensor ist.

11. System nach einem beliebigen der vorstehenden Ansprüche, **dadurch gekennzeichnet, dass** der externe Server oder zumindest einer der externen Server ein Wetterdienst-Server ist.

12. System nach einem beliebigen der vorstehenden Ansprüche, **dadurch gekennzeichnet, dass** der externe Server oder zumindest einer der externen Server eine mit von dem Benutzer des zweiten Endgeräts bereitgestellten Angaben erstellte psychologische Datenbank ist.

13. Vorrichtung zur mindestens einmaligen Prüfung einer Bedingung, dazu ausgelegt:
- Ein Nachrichtensystem zu befähigen, zumindest den Körper einer von einem anderen Endgerät gesendeten Nachricht an ein Endgerät zu übertragen, wenn das Ergebnis der Prüfung der Bedingung positiv ist;
- ein Nachrichtensystem daran zu hindern, zumindest den Körper einer von einem anderen Endgerät gesendeten Nachricht an ein Endgerät zu übertragen, wenn das Ergebnis der Prüfung der Bedingung negativ ist, zumindest während der Zeitspanne, während welcher das Ergebnis der Prüfung der Bedingung negativ bleibt;
**dadurch gekennzeichnet, dass** die Prüfvorrichtung weiterhin dazu ausgelegt ist:
- Über das Internet mit mindestens einem prüfvorrichtungsexternen Datenserver verbunden zu werden;
und dass die Vorrichtung zur Prüfung einer Bedingung ebenfalls dazu ausgelegt ist:
- Eine Nachricht, welche von einem Endgerät gesendet wurde und welcher mindestens eine Bedingung angehängt wurde, zu speichern;
- mindestens eine Information von mindestens einem externen Server zu empfangen;
- die Prüfung der der besagten gespeicherten Nachricht angehängten Bedingung mindestens teilweise anhand der von dem externen Server empfangenen Information durchzuführen.

14. Verfahren zur mindestens einmaligen Prüfung mindestens einer Bedingung, umfassend:
- Einen Schritt der Prüfung einer Bedingung, welcher es einem Nachrichtensystem im Fall eines positiven Ergebnisses der Prüfung der Bedingung ermöglicht, zumindest den Körper einer von einem anderen Endgerät gesendeten Nachricht an ein anderes Endgerät zu übertragen, und welcher ein Nachrichtensystem im Fall eines negativen Prüfergebnisses daran hindert, zumindest den Körper einer von einem Endgerät gesendeten Nachricht an ein anderes Endgerät zu übertragen, zumindest während der Zeitspanne, während welcher das Ergebnis der Prüfung der Bedingung negativ bleibt,
**dadurch gekennzeichnet, dass** das Prüfverfahren ebenfalls umfasst:
- Einen Schritt des Speicherns einer Nachricht, welche von einem Endgerät gesendet wurde und welcher mindestens eine Bedingung angehängt wurde;
- einen Schritt des Empfangens mindestens einer Information von mindestens einem externen Datenserver mittels einer Verbindung über das Internet;
und dass:
Der Schritt der Prüfung der der besagten gespeicherten Nachricht angehängten Bedingung zumindest teilweise anhand der von dem externen Server empfangenen Information durchgeführt wird.
